(19) Europäisches Patentamt
European Patent Office
Office européen des brevets

(11) EP 3 965 790 B1

(12) **FASCICULE DE BREVET EUROPEEN**

(45) Date de publication et mention de la délivrance du brevet:
**01.05.2024 Bulletin 2024/18**

(21) Numéro de dépôt: **20726028.2**

(22) Date de dépôt: **11.05.2020**

(51) Classification Internationale des Brevets (IPC):
***A61K 36/37** (2006.01)* ***A61K 31/56** (2006.01)*
***A61P 17/00** (2006.01)* ***A61K 31/192** (2006.01)*

(52) Classification Coopérative des Brevets (CPC):
**A61K 31/192; A61K 31/56; A61K 36/37; A61P 17/00**

(86) Numéro de dépôt international:
**PCT/EP2020/063036**

(87) Numéro de publication internationale:
**WO 2020/229403 (19.11.2020 Gazette 2020/47)**

(54) **TRITERPÈNES PENTACYCLIQUES DANS LE TRAITEMENT DU VITILIGO**

PENTACYCLISCHE TRITERPENE ZUR BEHANDLUNG VON VITILIGO

PENTACYCLIC TRITERPENES IN THE TREATMENT OF VITILIGO

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.05.2019 FR 1904890**

(43) Date de publication de la demande:
**16.03.2022 Bulletin 2022/11**

(73) Titulaire: **Pierre Fabre Dermo-Cosmétique**
**81500 Lavaur (FR)**

(72) Inventeurs:
- **NGUYEN, Thien**
  **31180 ROUFFIAC-TOLOSAN (FR)**
- **COUSY, Adrien**
  **31870 BEAUMONT SUR LEZE (FR)**

(74) Mandataire: **Regimbeau**
**20, rue de Chazelles**
**75847 Paris Cedex 17 (FR)**

(56) Documents cités:
**WO-A1-03/026603** **WO-A1-2017/194757**
**CN-A- 106 902 125**

- **CHANG Y ET AL: "Demethylzeylasteral inhibits the skin trafficking of CD8+ T cells in patients with vitiligo through the inactivation of IFN-[gamma]-JAK-STAT1-CXCL10 signaling pathway in keratinocytes", JOURNAL OF INVESTIGATIVE DERMATOLOGY 20180501 ELSEVIER B.V. NLD, vol. 138, no. 5, Supplement 1, 1 mai 2018 (2018-05-01), XP009518001, ISSN: 1523-1747**
- **XU WENYAN ET AL: "Tripterygium in dermatologic therapy", INTERNATIONAL JOURNAL OF DERMATOLOGY, WILEY-BLACKWELL PUBLISHING LTD, UK, vol. 24, no. 3, 1 avril 1985 (1985-04-01), pages 152-157, XP002650438, ISSN: 0011-9059**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### DOMAINE TECHNIQUE DE L'INVENTION

La présente invention concerne des compositions comprenant au moins un triterpène pentacyclique pour son utilisation dans le traitement du vitiligo.

### ETAT DE LA TECHNIQUE

Le vitiligo est une infection de la peau caractérisée par l'apparition de taches blanches sur les mains, les pieds, le visage ou toute autre partie du corps. Ces taches sont causées par une dépigmentation, c'est-à-dire la disparition des mélanocytes. La dépigmentation peut être plus ou moins importante et les taches blanches de tailles variables. Dans certains cas, les poils ou les cheveux qui poussent à l'intérieur des zones dépigmentées sont également blancs. Le vitiligo n'est ni contagieux, ni douloureux mais il peut entraîner une détresse psychologique importante. En effet, le vitiligo est souvent minimisé, il est encore insuffisamment pris en charge par les médecins. Les personnes ayant une peau foncée en souffrent tout particulièrement. Le vitiligo concerne environ 0,5 à 1% de la population mondiale, toute race ethnique confondue. Il apparaît généralement vers l'âge de 10 ans à 30 ans, la moitié des personnes atteintes le sont avant 20 ans. Le vitiligo est donc assez rare chez les jeunes enfants. Il touche aussi bien les hommes que les femmes.

Il existe plusieurs types de vitiligo :

- Le vitiligo segmentaire, localisé sur un seul côté du corps, par exemple sur une partie du visage, du haut du corps, d'une jambe ou d'un bras. Cette forme de vitiligo apparaît plus souvent chez les enfants ou les adolescents. La zone dépigmentée correspond à un territoire d'innervation, c'est-à-dire une zone de la peau innervée par un nerf en particulier. Cette forme apparaît rapidement en quelques mois, puis cesse généralement d'évoluer.
- Le vitiligo généralisé qui se présente sous forme de taches souvent plus ou moins symétriques, touchant les deux côtés du corps, en particulier des zones de frictions ou de pressions répétées. Le terme généralisé ne signifie pas forcément que les taches sont étendues. L'évolution est imprévisible, les taches pouvant rester petites et localisées ou s'étendre rapidement.
- Le vitiligo universalis, plus rare, qui s'étend rapidement et peut toucher presque la totalité du corps.

Le plus souvent, la maladie évolue à un rythme imprévisible et peut s'arrêter ou s'étendre sans que l'on sache pourquoi. Le vitiligo peut évoluer par phases, les aggravations survenant parfois après un évènement déclencheur psychologique ou physique. Dans de rares cas, les plaques disparaissent d'elles-mêmes. En dehors du préjudice esthétique, le vitiligo n'est pas une maladie grave. Les personnes atteintes de vitiligo ont toutefois un risque accru d'avoir un cancer de la peau, car les zones dépigmentées ne font plus barrière aux rayons du soleil. Ces personnes ont également une probabilité plus importante de souffrir d'autres maladies auto-immunes.

Les causes du vitiligo ne sont pas bien connues, on sait toutefois que l'apparition des taches blanches est due à la destruction des mélanocytes. Plusieurs hypothèses sont aujourd'hui avancées pour expliquer la destruction des mélanocytes. Le vitiligo est probablement une pathologie qui a à la fois des origines génétiques, environnementales et auto-immunes. Le vitiligo est une maladie à forte composante auto-immune. En effet, les personnes atteintes de vitiligo produisent des anticorps anormaux qui s'attaquent directement aux mélanocytes et contribuent à les détruire. Par ailleurs, le vitiligo est souvent associé à d'autres maladies auto-immunes, comme des troubles de la thyroïde, ce qui laisse supposer l'existence de mécanismes communs. Le vitiligo est également lié à des facteurs génétiques, qui n'ont pas tous été clairement identifiés. Il est fréquent que plusieurs personnes soient atteintes de vitiligo dans une même famille. Pas moins de dix gènes seraient impliqués. Selon plusieurs études, les mélanocytes des personnes atteintes de vitiligo accumuleraient de nombreux radicaux libres, ce qui entraînerait une autodestruction des mélanocytes.

Dans la littérature la plus récente, deux hypothèses attirent l'attention des chercheurs. La première se focalise sur la mort des mélanocytes qui serait due à des lymphocytes T CD8 « killer ». La seconde hypothèse est basée sur le détachement des mélanocytes, l'adhésion mélanocytes-kératinocytes assurée par la E-cadhérine est affectée lors de cette pathologie, cette protéine est spécifiquement dégradée par une métalloprotéase de type 9 (MMP-9). La MMP-9 est surproduite par les kératinocytes lorsqu'ils subissent un stress oxydatif, ou des inducteurs tels que les interleukines 17 (IL-17) provenant des lymphocytes T CD4 différenciés (Th17). La MMP-9 dégrade spécifiquement la protéine E-cadhérine et provoque la perte d'adhésion des mélanocytes sur les kératinocytes (Boukhedouni et al., Pigment Cell Melanoma Research, 31(6), P003, 756-757, 2018). Par ailleurs les IL-17 produites par les lymphocytes induisent les cytokines pro-inflammatoires et les chémiokines chez les kératinocytes, ces derniers exercent également un effet néfaste sur les mélanocytes qui se traduit par l'altération de leur fonction productive de mélanine (Kotobuki et al., Pigment Cell Melanoma Research, 25(2), 219-230, 2012).

A ce jour les traitements existants sont lourds. Il y a la photothérapie utilisant des UVB, un traitement local par

laser Excimer, le traitement chirurgical, les greffes cellulaires. Tous ces traitements ne sont pas sans risque, les effets secondaires peuvent être importants. De nouvelles voies de thérapies envisagées pour le vitiligo sont des molécules biologiques, administrées par voie injectable. Les anticorps capables d'inhiber spécifiquement la voie Th17 sont en cours d'essais cliniques. Les inconvénients existent, ce sont des traitements très coûteux et ne sont pas dénués d'effets secondaires et d'accoutumance.

Il existe donc une forte demande pour une thérapie plus efficace, sans effets secondaires et plus accessible au niveau du coût. La voie topique est une alternative, mais seules les petites molécules sont adaptées pour pouvoir passer dans l'épiderme et cibler les kératinocytes et les mélanocytes de manière efficace.

## RESUME DE L'INVENTION

La demanderesse propose des molécules « de novo » dans l'optique d'une formulation topique pour freiner et/ou stabiliser la progression voire réverter le vitiligo.

La présente invention concerne donc une composition comprenant la Tingénine A, la Tingénine B et le Célastrol ou leurs sels pharmaceutiquement acceptables pour son utilisation dans le traitement et/ou la prévention du vitiligo.

La présente divulgation porte, en outre, sur une méthode de traitement et/ou de prévention du vitiligo par administration à un patient en ayant besoin d'une quantité efficace d'une composition comprenant au moins un triterpène pentacyclique ou l'un de ses sels pharmaceutiquement acceptables.

La présente divulgation porte par ailleurs sur l'utilisation d'une composition comprenant au moins un triterpène pentacyclique ou l'un de ses sels pharmaceutiquement acceptables pour la fabrication d'un médicament pour le traitement et/ou la prévention du vitiligo.

La présente divulgation porte également sur l'utilisation d'une composition comprenant au moins un triterpène pentacyclique ou l'un de ses sels pharmaceutiquement acceptables pour le traitement et/ou la prévention du vitiligo.

## DESCRIPTION DETAILLEE DE L'INVENTION

### Définitions

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sel pharmaceutiquement acceptable » d'un composé, un sel qui est pharmaceutiquement acceptable, comme défini ici, et qui possède l'activité pharmacologique souhaitée du composé parent.

Les sels pharmaceutiquement acceptables comprennent notamment :

(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide2-naphtalènesulfonique, l'acide propionique, l'acide salicylique,l'acide succinique, l'acide dibenzoyl-L-tartrique, l'acide tartrique, l'acide p-toluènesulfonique, l'acide triméthylacétique, l'acide trifluoroacétique et similaires et ;

(2) les sels d'addition de base pharmaceutiquement acceptables formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, l'hydroxyde de sodium et similaires.

Il pourra s'agir d'un sel de sodium lorsque le composé comporte une fonction acide. Par « application topique », on entend une application sur la peau, les muqueuses et/ou les phanères.

Par « traitement » selon l'invention on entend l'inhibition et/ou la diminution de l'évolution ou du développement, plus particulièrement la régression, préférentiellement la disparition du vitiligo.

Par « prévention » selon l'invention, on entend empêcher, éviter ou retarder l'apparition du vitiligo.

Triterpènes pentacycliques

Les terpènes pentacycliques sont connus dans la phytothérapie pour traiter les maladies auto-immunes et certaines maladies inflammatoires comme le psoriasis, la dermatite atopique, l'arthrite rhumatoïde. Parmi eux, sont connus des triterpènes pentacycliques issus des plantes de la famille des Celastraceae (Tripterygium wilfordii, Tripterygium regeli, Tripterygium hydroglaucum, Celastrus paniculatus).

Par « triterpènes pentacycliques » selon l'invention, on entend les triterpènes pentacycliques naturellement produits par les cellules d'une plante de la famille des celastraceae et notamment la Tingénine A, encore appelée Tingénone ou Mayténine (de formule Chem. I ci-dessous), la Tingénine B, encore appelée 22 béta-hydroxy-tingénone (de formule Chem. II ci-dessous) et le Célastrol, encore appelé triptérine (de formule Chem. III ci-dessous).

[Chem. I]

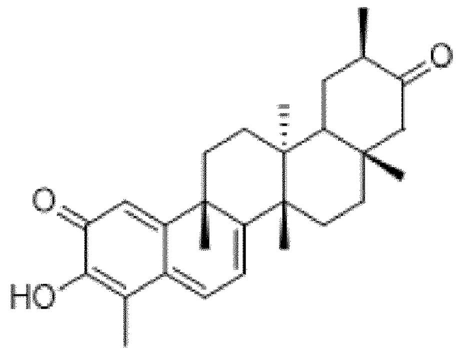

[Chem. II]

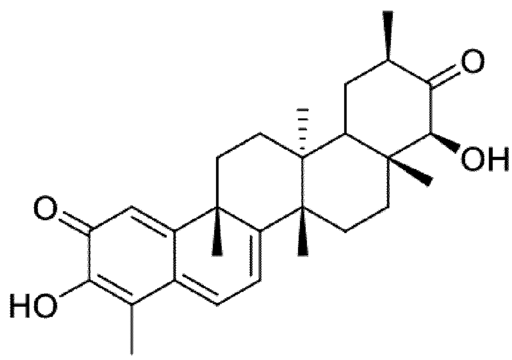

[Chem. III]

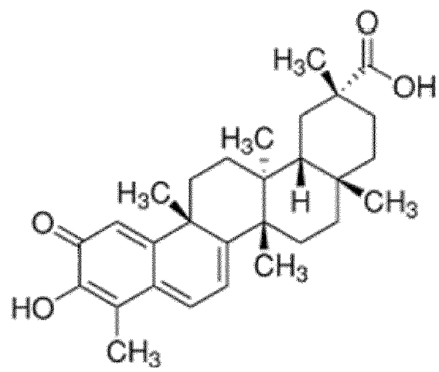

Dans un mode de réalisation, la composition pour une utilisation selon l'invention comprend la Tingénine A, la Tingénine B, le Célastrol et leurs sels pharmaceutiquement acceptables.

Dans un mode de réalisation, la composition selon l'invention peut comprendre au moins un triterpène pentacyclique ou l'un de ses sels pharmaceutiquement acceptables sous forme purifiée.

Par « forme purifiée » d'un triterpène pentacyclique ou l'un de ses sels pharmaceutiquement acceptables, on entend, au sens de la présente invention, un composé sous forme pure c'est-à-dire comprenant au moins 90% en poids, notamment au moins 95% en poids, notamment au moins 99% en poids du composé triterpène pentacyclique, notamment la Tingénine A, la Tingénine B et/ou le Célastrol, ou l'un de leurs sels pharmaceutiquement acceptables.

Dans un mode de réalisation, le ou les triterpène(s) pentacyclique(s) ou l'un de ses/leurs sels pharmaceutiquement acceptables peut/peuvent être présent(s) dans la composition selon l'invention sous la forme d'un extrait d'une plante de la famille des Celastraceae.

Par « plante de la famille des Celastraceae » selon l'invention, on entend l'ensemble des plantes appartenant à cette famille, et notamment les plantes du genre Tripterygium, Bhesa, Kokkona, Catha, Euonymus, Orthosphenia, Dicarpellum, Celastrus, Maytenus, Peritassa, Siphonodon et Rzedowskia, en particulier Tripterygium, Maytenus et Celastrus. Il s'agit de préférence de plantes telles que *Tripterygium wilfordii, Tripterygium regelii, Tripterygium hypoglaucum, Celastrus orbiculatus* ou *Maytenus ilicifolia,* notamment *Tripterygium wilfordii, Tripterygium hypoglaucum* ou *Celastrus orbiculatus* notamment *Tripterygium wilfordii.*

Dans un mode de réalisation cet extrait peut être brut ou enrichi en triterpène(s) pentacyclique(s), notamment en Tingénine A, Tingénine B et/ou célastrol. L'extrait peut alors comprendre éventuellement des composants (dont des composants actifs) issus de la plante autres que des triterpènes pentacycliques.

Par « extrait brut », on entend un extrait directement obtenu à partir des plantes.

Par « extrait brut enrichi », on entend un extrait dans lequel la quantité en triterpène(s) pentacyclique(s), notamment en Tingénine A, Tingénine B et/ou célastrol, est supérieure à 30% en poids, notamment supérieure à 50% en poids par rapport à la quantité en triterpènes pentacycliques dans un extrait brut.

Dans un mode de réalisation, l'extrait brut enrichi de la composition pour une utilisation selon l'invention comprend entre 90% et 100% de triterpène(s) pentacyclique(s) en poids par rapport au poids total de l'extrait brut enrichi.

Les extraits purifiés, bruts ou enrichis peuvent être obtenus à partir de n'importe quelle partie des plantes de la famille Celastraceae, notamment les racines, les graines et les parties aériennes.

Alternativement, ils peuvent être obtenus par des cellules de cultures de cellules végétales de ces plantes. Dans le cas de cultures de cellules végétales, cet extrait pourra notamment être obtenu à partir du surnageant, de la suspension ou de la biomasse desdites cultures cellulaires (tel que notamment décrit par Coppede et al., Plant Cell Tiss Organ Cuit, 118:33-43, 2014).

Dans un mode de réalisation, l'extrait brut enrichi est obtenu selon le procédé suivant :

(i) une phase de prolifération de cellules d'une plante de la famille des Celastraceae dans un milieu de prolifération,
(ii) une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) la préparation d'un extrait brut enrichi en triterpènes pentacycliques à partir de la culture cellulaire obtenue à l'étape (ii).

Par « cellules d'une plante de la famille des Celastraceae » selon l'invention, on entend les cellules de n'importe quelle partie de la plante : graines, racines, parties aériennes, et notamment des parties aériennes.

Par « parties aériennes » selon l'invention, on entend les parties de la plante situées au-dessus du sol, par exemple, les feuilles, les tiges, les pétioles et/ou les inflorescences, notamment les feuilles.

Par « phase de prolifération de cellules d'une plante de la famille des Celastraceae », on entend selon l'invention une phase dans laquelle les cellules d'une plante de la famille des Celastraceae sont en suspension dans un milieu de prolifération et dans des conditions adaptées à leur prolifération. Ces cellules pourront notamment être obtenues à partir de cals avant leur mise en suspension. Si nécessaire, les suspensions cellulaires pourront être régulièrement réensemencées afin de les maintenir dans des conditions de prolifération. Par « cal » selon l'invention, on entend un amas de cellules dédifférenciées, également appelées cellules souches ou cellules méristématiques.

L'induction de cals pourra être obtenue par toute méthode connue de l'homme du métier. Les cals selon l'invention pourront notamment être obtenus de la manière décrite ci-après. L'induction de cals à partir d'un explant de tissu de partie de plante, notamment une partie aérienne, de la famille des Celastraceae, par exemple Tripterygium wilfordii, est bien connue de l'homme du métier. L'induction de cals pourra notamment être réalisée par :

- obtention d'un explant de tissu de la plante, par exemple un morceau de feuille de taille de 1 $cm^2$ environ,
- mise en culture de l'explant sur un milieu de prolifération gélosé,
- incubation, notamment à l'obscurité, à une température d'environ 25-30°C.

*Etape (i) : phase de prolifération de cellules*

L'homme du métier connaissant les cultures de cellules d'une plante de la famille des Celastraceae pourra facilement déterminer la composition du milieu de prolifération nécessaire à leur prolifération. Préférentiellement, ce milieu de prolifération permettra la prolifération des cellules sous formes dédifférenciées, c'est-à-dire sous formes totipotentes. Le maintien sous forme dédifférenciée pourra notamment être obtenu par l'utilisation de ratios particuliers cytokinines/auxines dans le milieu de prolifération. Le milieu de prolifération pourra notamment comprendre :

- au moins un macroélément, notamment choisi parmi NH4NO3, KNO3, CaCl2.2H2O, MgSO4.7H2O, KH2PO4 et un mélange de ceux-ci, par exemple à une concentration en macroéléments totale comprise entre 1000 et 9000 mg/L, par exemple entre 3000 et 8000 mg/L de milieu de prolifération : lors de la phase de prolifération, la concentration en macroéléments totale du milieu de prolifération sera notamment comprise entre 3000 et 5500 mg/L de milieu de prolifération ;
- au moins un microélément, notamment choisi parmi KI, H3BO3, MnSO4.4H2O, ZnSO4.H2O, Na2MoO4.2H2O, CuSO4.5H2O, CoCl2.6H2O, FeSO4.7H2O, Na2EDTA.2H2O et un mélange de ceux-ci, par exemple à une concentration en microéléments totale comprise entre 10 et 200 mg/L, notamment entre 50 et 150 mg/L de milieu de prolifération ;
- au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3 g/L, notamment de 0,05 à 1 g/L de milieu de prolifération ;
  au moins un acide aminé, notamment la glycine, par exemple à une concentration en acide aminés totale pouvant aller de 0,15 à 5 mg/L, notamment entre 1 et 4 mg/L de milieu de prolifération : lors de la phase de prolifération, la concentration en acide aminés du milieu de prolifération sera notamment comprise entre 1 et 2,5 mg/L de milieu prolifération ;
- au moins une source de carbone, notamment du saccharose, par exemple à une concentration totale en source de carbone de 10 à 70 g/L de milieu de prolifération, par exemple à environ 30 g/L ;
- au moins une hormone végétale (encore appelée hormone de croissance végétale ou facteur de croissance végétale ou régulateur de croissance végétale) notamment choisie parmi une ou plusieurs cytokinines, notamment la kinétine et/ou la 6-furfurylaminopurine, une ou plusieurs auxines, notamment l'acide 2,4-dichlorophénoxyacétique (2,4D) et/ou l'acide naphthalène acétique (NAA), et un mélange de celles-ci. Lors de la phase de prolifération le milieu de prolifération comprendra notamment au moins une cytokinine et au moins une auxine.

Les hormones de croissance végétale seront notamment ajoutées au milieu de prolifération à une concentration et à un ratio permettant la prolifération des cellules sous formes dédifférenciées. Elles seront notamment choisies parmi la kinétine, la 6-furfurylaminopurine, l'acide 2,4-dichlorophénoxyacétique (2,4D), l'acide naphthalène acétique (NAA) et un mélange de ceux-ci ; notamment choisies parmi la kinétine, l'acide 2,4-dichlorophénoxyacétique (2,4D), l'acide naphthalène acétique (NAA) et un mélange de ceux-ci. Il pourra s'agir en particulier d'un mélange de kinétine, acide 2,4-dichlorophénoxyacétique (2,4D) et acide naphthalène acétique.

La concentration en auxines sera notamment comprise entre 0,001 et 10 mg/L de milieu de prolifération, par exemple entre 0,1 et 3 mg/L de milieu de prolifération.

La concentration en cytokines sera notamment comprise entre 0,01 et 0,5 mg/L de milieu de prolifération, par exemple entre 0,05 et 0,15 mg/L de milieu de prolifération.

Dans un mode de réalisation, le ratio hormonal auxines / cytokinines sera compris entre 0,2 à 2,5/0,01 à 0,5, notamment entre 1 à 2/ 0,05 à 0,2 notamment sera d'environ 1,5/0,1. Notamment le milieu de de prolifération selon l'invention pourra comprendre 1,5 mg/L d'auxine, notamment d'acide 2,4-dichlorophénoxyacétique (2,4D) et d'acide naphthalène acétique (NAA), et 0,1 mg/L de cytokinine, notamment de kinétine.

Le milieu de prolifération sera stérile et préférentiellement à un pH proche de la neutralité. Un exemple de milieu de prolifération adapté à la prolifération des cellules d'une plante de la famille des Celastraceae selon l'invention est notamment décrit par Murashige & Skoog (1962) ou dans les exemples de la présente demande.

Ce milieu de prolifération peut par exemple avoir la composition suivante (les concentrations sont exprimées par rapport au volume de milieu de prolifération sans cellules) : Macroéléments : NH4NO3 à 1 650 mg/L, KNO3 à 1 900 mg/L, CaCl2.2H2O à 440 mg/L, MgSO4.7H2O à 370 mg/L, KH2PO4 à 170 mg/L ; Microéléments : KI à 0,83 mg/L, H3BO3 à 6,2 mg/L, MnSO4.4H2O à 22,3 mg/L, ZnSO4.H2O à 6,6 mg/L, Na2MoO4.2H2O à 0,25 mg/L, CuSO4.5H2O à 0,025 mg/L, CoCl2.6H2O à 0,025 mg/L, FeSO4.7H2O à 27,8 mg/L, Na2EDTA.2H2O à 37,3 mg/L ; Vitamines : myo-inositol à 100 mg/L, acide nicotinique à 0,5 mg/L, pyridoxine-HCl à 0,5 mg/L, thiamine-HCl à 0,5 mg/L ; Acides aminés : glycine à 2 mg/L ; Source de carbone : saccharose à 30 g/L ; et Hormones végétales : acide naphtalène acétique à 1 mg/L, acide 2,4-dichlorophénoxyacétique à 0,5 mg/L, kinétine à 0,1 mg/L, le tout ajusté à pH 6 avant stérilisation (par exemple par autoclavage de 20 min à 121°C ou par filtration sur filtre de 0,2 $\mu$m).

Alternativement, le milieu de prolifération peut avoir la composition suivante (les concentrations sont exprimées par rapport au volume de milieu de prolifération sans cellules) : Macroéléments : $NH_4NO_3$ à 1650 mg/L, $KNO_3$ à 2500 mg/L, $CaCl_2.2H_2O$ à 440 mg/L, $MgSO_4.7H_2O$ à 370 mg/L, $KH_2PO_4$ à 130 mg/L ; Microéléments : KI à 0,41 mg/L, $H_3BO_3$ à 6,2 mg/L, $MnSO_4.4H_2O$ à 22,3 mg/L, $ZnSO_4.H_2O$ à 7,5 mg/L, $Na_2MoO_4.2H_2O$ à 0,25 mg/L, $CuSO_4.5H_2O$ à 0,025 mg/L, $CoCl_2.6H_2O$ à 0,025 mg/L, $FeSO_4.7H_2O$ à 19,85 mg/L, $Na_2EDTA.2H_2O$ à 26,64 mg/L ; Vitamines : myo-inositol à 50 mg/L, acide nicotinique à 0,25 mg/L, pyridoxine-HCl à 0,25 mg/L, thiamine-HCl à 0,25 mg/L ; Source de carbone : saccharose à 30 g/L ; et Hormones végétales : kinétine à 0,083 mg/L, acide 2,4-dichlorophénoxyacétique (2,4D) à 0,575 mg/L, acide naphthalène acétique (NAA) à 0,350 mg/L.

Alternativement, le milieu de prolifération peut avoir la composition suivante (les concentrations sont exprimées par rapport au volume de milieu de prolifération sans cellules) : $NH_4NO_3$ à 20 mM, $KNO_3$ à 19 mM, $CaCl_2.2H_2O$ à 3 mM, $MgSO_4.7H_2O$ à 1,50 mM, $KH_2PO_4$ à 1,2 mM, KI à 0,005 mM, $H_3BO_3$ à 0,1 mM, $MnSO_4.4H_2O$ à 0,1 mM, $ZnSO_4.H_2O$ à 0,04 mM, $Na_2MoO_4.2H_2O$ à 0,001 mM $CuSO_4.5H_2O$ à 0,0001 mM, $CoCl_2.6H_2O$ à 0,0001 mM, $FeSO_4.7H_2O$ à 0,1 mM, $Na_2EDTA.2H_2O$ à 0,1 mM, myo-inositol 0,5 mM, acide nicotinique à 0,004 mM, pyridoxine-HCl à 0,002 mM, thiamine-HCl à 0,0015 mM, glycine à 0,03 mM, saccharose à 87,6 mM, acide naphtalène acétique à 0,005 mM, acide 2,4-dichlorophénoxyacétique à 0,002 mM, et kinétine à 0,0005 mM.

L'ensemencement du milieu de prolifération pourra être réalisé à partir de la mise en suspension de cellules de cals à une concentration comprise entre 20 et 300 g dans 1L de milieu de prolifération et préférentiellement entre 100 et 200 g dans 1L de milieu de prolifération, par exemple environ 150 g dans 1L de milieu de prolifération. La phase de prolifération aura lieu dans des conditions de multiplication de la biomasse.

Par « conditions de multiplication de la biomasse » selon l'invention, on entend notamment les conditions de température, de durée, d'agitation et de luminosité nécessaires à la prolifération des cellules en suspension. L'homme du métier connaissant les cultures de cellules d'une plante de la famille de Celastraceae pourra facilement déterminer les conditions de multiplication de la biomasse. Dans un mode de réalisation selon l'invention, l'étape de prolifération de la biomasse se fera à l'obscurité, à une température comprise entre 20 et 35 °C, notamment entre 27 et 28°C, notamment à environ 27 ou 28°C, en particulier sous une agitation comprise entre 100 et 200 rpm, notamment à environ 125 rpm (orbitale de 22,5 mm) et pendant une durée comprise entre 10 et 30 jours, notamment 15 jours de culture.

Au cours de cette étape, les cellules peuvent être « repiquées » ou propagées, par exemple tous les 7 à 15 jours. Le repiquage des cellules est bien connu par l'homme du métier, il pourra notamment consister à diluer une partie de la culture cellulaire dans du milieu neuf concentré. Par exemple, 1/5ème de la culture est remise en suspension dans un volume de milieu neuf correspondant au volume de la culture initiale. Il permet l'entretien de la lignée cellulaire en milieu liquide dans un état de prolifération.

*Etape (ii) : phase d'élicitation*

Après la phase de prolifération, les cellules obtenues dans la phase (i) sont élicitées par ajout d'un cocktail d'élicitation. Le milieu de prolifération auquel a été ajouté le cocktail d'élicitation sera nommé « milieu d'élicitation ». La phase d'élicitation est la phase dans laquelle les cellules sont maintenues dans un état physiologique favorisant la biosynthèse de métabolites secondaires tels que les triterpènes pentacycliques.

La production de triterpènes pentacycliques, a lieu, au cours de la phase d'élicitation, dans le cytosol de la cellule et peut diffuser en partie dans le milieu d'élicitation. La phase d'élicitation au sens de la présente invention correspond donc à la phase de production (biosynthèse) des triterpènes pentacycliques et plus particulièrement du Célastrol, de la Tingénine A et de la Tingénine B.

L'élicitation se fera préférentiellement après une phase de prolifération de 7 à 21 jours, notamment 12 à 20 jours, notamment 15, 16 ou 17 jours (notamment sans repiquage). Alternativement l'ajout du cocktail d'élicitation pourra se faire lorsque la concentration en cellules obtenue lors de la phase de prolifération est double, notamment supérieure au double, par rapport à la concentration initiale en cellules dans le milieu de prolifération. L'ajout du cocktail d'élicitation pourra notamment se faire lorsque la concentration en cellule est supérieure à 200 g/L, par exemple entre 200 et 400 g/L, notamment environ 300 g/L (en quantité de cellules par litre de milieu de prolifération). Dans un mode de réalisation, l'ajout du cocktail d'élicitation se fera dans une culture dont la concentration en cellules est passée de 150 à 200 g/L lors du début de la phase de prolifération à une concentration comprise entre 300 et 400 g/L lors de la fin de la phase de prolifération.

A la suite de la phase de prolifération, les cellules végétales ont consommé la plupart des, voire tous les éléments contenus dans le milieu de prolifération, et en particulier les sources de carbone telles que le saccharose. Il peut donc être nécessaire de rétablir la composition du milieu avant ou en même temps que l'ajout du cocktail d'élicitation.

Pour rétablir la composition du milieu, on peut notamment concentrer la culture cellulaire obtenue après l'étape de prolifération, par exemple par décantation ou filtration puis ajouter du milieu de prolifération neuf aux cellules ainsi obtenues. Dans ce cas, les cellules seront re-suspendues dans le milieu de prolifération neuf de manière à obtenir une concentration comprise entre 200 g/L et 400 g/L, par exemple entre 250 et 350 g/L, notamment d'environ 300 g/L (en

quantité de cellules par litre de milieu de prolifération).

Alternativement, on peut ajouter dans la culture cellulaire un mélange concentré permettant de rétablir les concentrations des éléments du milieu de prolifération. Le mélange concentré sera notamment ajouté juste avant, juste après ou en même temps que le cocktail d'élicitation. Par exemple on peut remplacer 1/5$^{ème}$ de la culture cellulaire par un volume équivalent du milieu de prolifération concentré 5 fois.

On considère que la concentration en éléments du milieu de prolifération est proche ou équivalente à zéro après 14, 15 ou 16 jours de phase de prolifération. En particulier on considère que la concentration en éléments minéraux (plus particulièrement les macroéléments et les microéléments) et carbonés (plus particulièrement les sources de carbone) est proche de ou équivalente à zéro après 14, 15 ou 16 jours de phase de prolifération.

Dans un mode de réalisation, le milieu de prolifération selon l'invention au début de la phase d'élicitation comprendra entre autres :

- au moins un macroélément, notamment choisi parmi NH4NO3, KNO3, CaCl2.2H2O, MgSO4.7H2O, KH2PO4 et un mélange de ceux-ci, par exemple à une concentration de macroéléments totale comprise entre 5 000 et 8 000 mg/L, préférentiellement supérieure à 6 000 mg/L de milieu de prolifération, avantageusement entre 6 000 et 8 000 mg/L ;
- au moins un microélément, notamment choisi parmi KI, H3BO3, MnSO4.4H2O, ZnSO4.H2O, Na2MoO4.2H2O, CuSO4.5H2O, CoCl2.6H2O, FeSO4.7H2O, Na2EDTA.2H2O et un mélange de ceux-ci, par exemple à une concentration en microéléments totale comprise entre 10 et 200 mg/L, notamment entre 50 et 150 mg/L de milieu prolifération,
- au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3 g/L, notamment de 0,05 à 1 g/L de milieu de prolifération ;
- au moins un acide aminé, notamment la glycine, par exemple à une concentration dans le milieu de prolifération comprise entre 3 et 4 mg/L de milieu de prolifération ;
- au moins une source de carbone, notamment du saccharose, par exemple à une concentration totale en source de carbone de 10 à 70 g/L de milieu de prolifération, par exemple à environ 30 g/L. Dans un mode de réalisation le milieu de prolifération au début de la phase d'élicitation ne comprendra pas, où comprendra une quantité négligeable, notamment moins de 0,001 g/ml, de cytokinine et d'auxine. Le milieu de prolifération lors de la phase d'élicitation sera avantageusement stérile et préférentiellement à un pH proche de la neutralité.

Le milieu de prolifération lors de la phase d'élicitation pourra notamment avoir la composition suivante : Macroéléments : NH4NO3 à 2,8 g/L, KNO3 à 3 g/L, CaCl2.2H2O à 0,45 g/L, MgSO4.7H2O à 74 mg/L, KH2PO4 à 34 mg/L ; Microéléments : KI à 0,16 mg/L, H3BO3 à 6.2 mg/L, MnSO4.4H2O à 18,5 mg/L, ZnSO4.H2O à 6,6 mg/L, Na2MoO4.2H2O à 0,25 mg/L, CuSO4.5H2O à 0,025 mg/L, CoCl2.6H2O à 0,025 mg/L, FeSO4.7H2O à 28 mg/L, Na2EDTA.2H2O à 37 mg/L ; Vitamines : myo-inositol à 250 mg/L, acide nicotinique à 1,7 mg/L, pyridoxine-HCl à 1 mg/L, thiamine-HCl à 1 mg/L ; Acide aminé : glycine à 4 mg/L ; Source de carbone : saccharose à 30 g/L.

Par « cocktail d'élicitation » selon l'invention, on entend un cocktail permettant de stopper la division cellulaire. Ce cocktail d'élicitation comprend au moins un éliciteur de type composé monocarboxylique et au moins un éliciteur biotique. Le cocktail d'élicitation est introduit, par exemple, à l'aide de solutions mères concentrées dans le milieu de culture. Par « éliciteur de type composé monocarboxylique » selon l'invention, on entend plus particulièrement un éliciteur choisi dans le groupe comprenant, de préférence constitué par, l'acide 5-chloro salicylique (5-Chloro SA), l'acide salicylique (SA), l'acide acétyl-salicylique (ASA), un ester de méthyle, notamment le j asmonate de méthyle (MeJa), et un mélange de ceux-ci. Dans un mode de réalisation selon l'invention, l'éliciteur de type composé monocarboxylique est le jasmonate de méthyle, l'acide salicylique et/ou l'acide 5-chloro salicylique, notamment le jasmonate de méthyle. L'éliciteur de type composé monocarboxylique sera notamment ajouté de façon à obtenir une concentration finale comprise entre 0,005 et 0,1 g/L, notamment 0,01 et 0,05 g/L de milieu d'élicitation, notamment de 0,002 et 0,004 g/L de milieu d'élicitation.

Par « éliciteur biotique » selon l'invention, on entend plus particulièrement un éliciteur biotique choisi dans le groupe comprenant, de préférence constitué par, une N-acétylaminoglucosamine, notamment la chitine, le chitosan, les extraits de microorganismes ou de champignons, les oligosaccharides (polysaccharides, pectines, cellulose). Dans un mode de réalisation, l'éliciteur biotique est la chitine. La chitine est un polymère linéaire de motif répétitif : béta-1,4 N-acétyl D-glucosamine. L'éliciteur biotique sera notamment ajouté de façon à obtenir une concentration finale comprise entre 0,05 et 50 g/L de milieu d'élicitation, par exemple de 0,1 à 10 g/L, par exemple de 0,5 à 7 g/L, notamment de 1 à 5 g/L de milieu de d'élicitation.

Dans un mode de réalisation, le cocktail d'élicitation comprend du jasmonate de méthyle, notamment à une concentration finale dans le milieu d'élicitation entre 0,002 et 0,005 g/L, et de la chitine, notamment à une concentration finale entre 1 et 4 g/L de milieu d'élicitation.

Dans un mode de réalisation, le cocktail d'élicitation selon l'invention comprendra en outre, au moins un facteur de différenciation cellulaire des cellules végétales et/ou au moins un précurseur de la voie de synthèse des terpènes.

Le « facteur de différenciation cellulaire des cellules végétales » selon l'invention pourra notamment être choisi dans le groupe comprenant, de préférence constitué par, une cytokinine, notamment la benzyl-aminopurine (BAP), l'acide abscissique, la kinétine, le thidiazuron, la 6-y,y-diméthylallylaminopurine (2iP ou isopentényladénine) ou la zéatine, une gibbérelline et un mélange de celles-ci, notamment la BAP et/ou la 2iP, en particulier la 2iP.

Le « précurseur de synthèse des terpènes » selon l'invention pourra notamment être choisi dans le groupe comprenant, de préférence constitué par, le pyruvate de sodium ; le pyrophosphate de potassium ; l'acide mévalonique ; le géraniol ; le farnésol ; l'isopenténylе, le diméthylallyle, y compris leurs formes pyrophosphatées ; l'acétate de sodium ; l'acide pyruvique et leurs mélanges, notamment le géraniol, le farnésol, le pyruvate de sodium, le pyrophosphate de potassium et leurs mélanges, tel que le pyruvate de sodium et/ou le pyrophosphate de potassium.

La BAP pourra notamment être utilisée à une concentration finale dans le milieu d'élicitation comprise entre 0,01 et 5 mg/L, par exemple entre 0,5 et 5 mg/L de milieu d'élicitation.

L'acide 5-chloro salicylique (5-Chloro SA) pourra notamment être utilisé à une concentration finale dans le milieu d'élicitation comprise entre 0,1 et 15 mg/L.

L'acide salicylique pourra notamment être utilisé à une concentration finale dans le milieu d'élicitation comprise entre 0,1 et 100 mg/L, par exemple entre 20 et 60 mg/L, par exemple environ 45 mg/L.

Le farnésol pourra notamment être utilisé à une concentration finale dans le milieu d'élicitation de 1 à 100 mg/L, par exemple de 15 à 30 mg/L, par exemple à environ 30 mg/L.

Le géraniol pourra notamment être utilisé à une concentration finale dans le milieu d'élicitation de 1 à 100 mg/L, par exemple de 20 à 30 mg/L.

Le pyruvate de sodium pourra notamment être utilisé à une concentration finale dans le milieu d'élicitation de 100 à 5 000 mg/L, par exemple de 500 à 2 000 mg/L.

Le pyrophosphate de potassium pourra notamment être utilisé à une concentration finale dans le milieu d'élicitation de 1 à 2 000 mg/L, par exemple de 100 à 1 000 mg/L de milieu d'élicitation.

La 2iP pourra notamment être utilisée à une concentration finale dans le milieu d'élicitation de 0,005 à 10mg/L, par exemple de 0,01 mg/L à 3 mg/L, par exemple de 0,1 à 2 mg/L. Dans un mode de réalisation de l'invention, le cocktail d'élicitation comprend du jasmonate de méthyle, de la chitine, du pyruvate de sodium, du pyrophosphate de potassium ou un mélange de ceux-ci.

Dans un mode de réalisation de l'invention, le cocktail d'élicitation comprend du jasmonate de méthyle, de la chitine, du pyruvate de sodium, du pyrophosphate de potassium, et éventuellement de la BAP et/ou de la 2iP.

Dans un mode de réalisation de l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2 000 mg/L), du pyrophosphate de potassium (de 100 à 1 000 mg/L), de la 2iP (de 0,1 à 2 mg/L), du j asmonate de méthyle (de 0,002 à 0,005 g/L) et de la chitine (de 1 à 4 g/L).

Dans un autre mode de réalisation, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) de la benzyl-aminopurine (BAP) (de 0,5 à 5 mg/L, notamment de 0,5 à 3 mg/L) ; de l'acide 5-chloro salicylique (5-Chloro SA) (de 2 à 6 mg/L, notamment de 3 à 5 mg/L, par exemple à environ 3 ou à environ 5 mg/L), de l'acide acétyl-salicylique (ASA) et/ou de l'acide salicylique (SA) (de 20 à 60 mg/L, notamment de 30 à 50 mg/L, notamment de 33 à 45 mg/L) ; du jasmonate de méthyle (MeJA) (de 0,002 à 0,005 g/L, notamment de 10 à 40 mg/L) ; de la chitine (de 1 à 4 g/L) ; et du farnésol (F-OH) (de 19 à 40 mg/L) et/ou du géraniol (de 20 à 30 mg/L).

Dans un autre mode de réalisation le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2 000 mg/L), du pyrophosphate de potassium (de 100 à 1 000 mg/L), de la 2iP (de 0,1 à 1 mg/L), du jasmonate de méthyle (MeJA) (de 0,002 à 0,005 g/L, notamment de 10 à 40 mg/L) et de la chitine (de 1 à 4 g/L). Dans un autre mode de réalisation le cocktail d'élicitation comprend ou consiste en (les concentrations données entre parenthèses correspondent à la concentration dans le milieu d'élicitation, le cocktail pouvant être plus ou moins concentré en fonction de la dilution envisagée) du pyruvate de sodium (environ 1,5 g/L), du pyrophosphate de potassium (environ 0,4 g/L), du 2iP (environ 0,4 mg/L), du jasmonate de méthyle (MeJA) (environ 0,03 mg/L), de la chitine (environ 2 g/L).

Pendant la phase d'élicitation, après ajout du cocktail d'élicitation, la culture est maintenue sous agitation, notamment entre 50 et 200 rpm, notamment à environ 125 rpm, pendant une période comprise entre 3 et 30 jours, notamment entre 10 et 25 jours, notamment de 12 à 15 jours, en particulier à une température comprise entre 20 et 35 °C, notamment à environ 27 °C et avantageusement avec un taux d'oxygène dissout dans le milieu de culture de 2 à 40 %, préférentiellement à environ 16 %. Un apport en air stérile suffisamment enrichi en oxygène pourra être fourni si

nécessaire notamment dans le volume mort du bioréacteur ou par diffusion dans le milieu. De préférence, la phase d'élicitation (iii) est menée à l'obscurité. Lors de la phase d'élicitation, la culture cellulaire n'est préférentiellement pas repiquée.

*Etape (iii) : phase de préparation de l'extrait brut enrichi en triterpènes pentacycliques*

Après la phase d'élicitation, le procédé comprend une étape de préparation d'un extrait brut enrichi en triterpènes pentacycliques.

L'extrait brut enrichi pourra notamment être obtenu par séparation de la biomasse et du surnageant de culture. La séparation pourra notamment être faite par filtration directe (0-50 $\mu$m), par centrifugation ou par décantation des cellules.

Dans un mode de réalisation, l'extrait brut enrichi compris dans la composition pour une utilisation selon l'invention pourra consister en le surnageant de culture ainsi récupérée.

L'extrait brut enrichi pourra également être obtenu après la lyse de la biomasse. Par exemple, les cellules contenues dans la biomasse récupérée pourront être lysées par une méthode physique (sonication ou broyage) ou chimique (lyse acide) puis ce lysat sera soumis à une extraction par solvant. La phase organique, comprenant notamment les triterpènes issus du cytosol, est ensuite récupérée, notamment par décantation ou centrifugation. Le solvant sera notamment un solvant de type ester, et plus particulière un acétate d'alkyle, l'alkyle étant plus particulièrement linéaire ou ramifié à 1 à 6 atomes de carbone, notamment l'acétate d'éthyle ou l'acétate d'isopropyle. Le volume de solvant utilisé sera notamment de 2 volumes par poids de biomasse.

Préférentiellement, l'extrait brut enrichi de la composition pour une utilisation selon l'invention correspondra à la phase organique ainsi récupérée.

Alternativement, l'extrait brut enrichi pourra être obtenu après évaporation du solvant et sa substitution notamment par un support adapté au domaine d'utilisation de l'extrait (cosmétique, pharmaceutique) et notamment les huiles végétales ou des solvants tel que notamment le Pentylène Glycol (ou Pentiol), ou du Myritol 318®.

L'extrait brut enrichi n'est pas purifié. L'extrait enrichi brut pourra éventuellement être purifié en une étape ultérieure.

Lorsque l'extrait brut enrichi est purifié, pour donner un extrait purifié, le procédé selon l'invention comprend en outre une étape (iv) d'obtention d'un extrait purifié d'un ou plusieurs triterpènes pentacycliques à partir de l'extrait enrichi brut obtenu à l'étape (iii). L'extrait purifié selon l'invention comprendra alors plus de 90 %, notamment plus de 95 %, notamment plus de 98 % en poids d'un ou plusieurs triterpènes pentacycliques selon l'invention par rapport au poids total de l'extrait.

Dans un mode de réalisation, l'extrait purifié selon l'invention comprend 60 % ou plus, notamment 80% ou plus, notamment 90% ou plus, notamment 95 % ou plus, notamment plus de 98 % ou plus, notamment 100 % de Célastrol en poids par rapport au poids total de l'extrait. Il pourra comprendre notamment entre 50% et 98%, notamment entre 70% et 90%, notamment entre 76% et 84% de Célastrol en poids par rapport au poids total de l'extrait.

Dans un mode de réalisation, l'extrait purifié selon l'invention comprend entre 1 et 20%, notamment entre 5 et 15 %, notamment entre 8 et 12 % de Tingénine A en poids par rapport au poids total de l'extrait.

Dans un mode de réalisation, l'extrait purifié selon l'invention comprend entre 1 et 20 %, notamment entre 5 et 15 %, notamment entre 8 et 12 % de Tingénine B en poids par rapport au poids total de l'extrait.

Dans un mode de réalisation, l'extrait purifié selon l'invention comprend :

- entre 50% et 98%, notamment entre 70% et 90%, notamment entre 76% et 84% de Célastrol en poids par rapport au poids total de l'extrait ;
- entre 1 et 20%, notamment entre 5 et 15 %, notamment entre 8 et 12 % de Tingénine A en poids par rapport au poids total de l'extrait ; et
- entre 1 et 20 %, notamment entre 5 et 15 %, notamment entre 8 et 12 % de Tingénine B en poids par rapport au poids total de l'extrait.

La purification de l'extrait enrichi selon l'invention pourra notamment être réalisée par une phase de séparation du ou des triterpène(s) pentacyclique(s), notamment le Célastrol, la Tingénine A et/ou la Tingénine B, notamment par fractionnement HPLC (chromatographie liquide à haute performance), au cours duquel les pics à 426 nm comprenant le Célastrol, la Tingénine A et la Tingénine B sortent respectivement à 19,75 min, 18,03 min et 16,21 min.

Compositions

Dans un mode de réalisation, la composition pour une utilisation selon l'invention comprend du célastrol entre 0,002 et 10% en poids par rapport au poids de la composition totale, notamment entre 0,01 et 2%, notamment entre

0,1 et 1%.

Dans un mode de réalisation, la composition pour une utilisation selon l'invention comprend de la Tingénine A entre 0,002 et 10% en poids par rapport au poids de la composition totale, notamment entre 0,01 et 2%, notamment entre 0,1 et 1%.

Dans un mode de réalisation, la composition pour une utilisation selon l'invention comprend de la Tingénine B entre 0,002 et 10% en poids par rapport au poids de la composition totale, notamment entre 0,01 et 2%, notamment entre 0,1 et 1%.

Dans un mode de réalisation, la composition pour une utilisation selon l'invention comprend entre 0,002 et 10% en poids de triterpène(s) pentacycliques(s) par rapport au poids de la composition totale, notamment entre 0,01 et 2%, notamment entre 0,1 et 1%, le ou les triterpène(s) pentacyclique(s) étant en particulier choisi parmi le Célastrol, la Tingénine A, la Tingénine B et leurs sels pharmaceutiquement acceptables.

Dans un mode de réalisation, la composition pour une utilisation selon l'invention comprend entre 0,002 et 10% d'un extrait brut enrichi en triterpène(s) pentacyclique(s) en poids par rapport au poids de la composition totale, notamment entre 0,01 et 2%, notamment entre 0,1 et 1%.

Dans un mode de réalisation, la composition pour une utilisation selon l'invention comprend entre 0,5 et 2% de Célastrol, entre 0,01% et 1% de Tingénine A et/ou entre 0,01% et 1% de Tingénine B. Dans un mode de réalisation, la composition pour une utilisation selon l'invention comprend entre 0,5 et 2% de Célastrol, entre 0,01% et 1% de Tingénine A et entre 0,01% et 1% de Tingénine B.

La composition pour son utilisation selon l'invention pourra en outre comprendre un ou plusieurs excipients pharmaceutiquement acceptables.

L'invention vise de préférence des compositions selon l'invention se présentant sous une forme propre et adaptée à une application topique.

Les compositions selon l'invention pourront se présenter ainsi sous les formes qui sont habituellement connues pour une administration topique, c'est-à-dire notamment les lotions, les shampoings, les baumes, les mousses, les gels, les dispersions, les émulsions, les sprays, les sérums, les masques ou les crèmes, avec des excipients permettant notamment une pénétration afin d'améliorer les propriétés et l'accessibilité des principes actifs.

Ces compositions contiennent généralement, outre le ou les triterpène(s) pentacyclique(s) selon l'invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents hydratants ou des eaux thermales, etc.

Ces compositions selon l'invention peuvent être utilisées quotidiennement, une à plusieurs fois par jour, sans limitation de durée de traitement.

## FIGURE

[Fig. 1] : chromatogramme HPLC de l'extrait CCV.

## EXEMPLES

Exemple 1 : obtention de l'extrait de culture de cellules végétales CCV de *Tripterygium wilfordii* enrichi en triterpènes pentacycliques

On réalise une culture dans un réacteur de type Wave (volume 5 L) Sartorius Stedim Biotech (Germany). On inocule le réacteur avec une suspension de cellules de *Tripterygium wilfordii* issus d'un Erlenmeyer (composition milieu, paramètres cités plus haut). Sous agitation en continu, après avoir atteint le maximum en biomasse environ 17 jours après, on procède à l'élicitation avec le cocktail éliciteur (jasmonate de méthyle et chitine). On arrête la culture après 15 jours d'élicitation. La majorité de la biomasse est récupérée par filtration de la suspension cellulaire avec un filtre nylon (20-50 $\mu$m). A partir de 5 L de suspension, on récupère environ 1925 g de biomasse. Cette biomasse est extraite avec de l'acétate d'éthyle (ou encore de l'acétate d'isopropyle) en proportion 2 : 1 (Volume : Poids) par rapport au poids de biomasse (ici 3850 mL de solvant pour 1925 g de biomasse). Le mélange biomasse/solvant est alors soumis à une extraction physique, par sonication ou par broyage. La phase organique est alors récupérée après macération sous agitation. L'ajout du solvant (suivi d'une macération sous agitation et de la récupération de la phase organique) est répété deux fois. On peut concentrer le solvant sous vide, puis solubiliser ensuite dans du pentylène glycol par exemple, et finir l'étape en tirant sous vide afin d'éliminer le solvant organique résiduel. On obtient alors une solution nommée « extrait CCV » contenant les triterpènes pentacycliques suivants : Célastrol (pic majeur), Tingénine A et Tingénine B (voir Figure 1).

Conditions chromatographie HPLC : appareil de chromatographie liquide Alliance (Waters 2695 version 2.03) ;

Colonne Sunfire C18, 100Å, 5 μm (4.6 mm X 150 mm). Gradient solvants : eau/acétonitrile, débit 3 ml/min. détection des triterpènes à λ 460 nm. Cet extrait utilisé pour les activités pharmacologiques (exemples 2 et 3) est titré en concentration en Célastrol. On peut purifier le Célastrol, la Tingénine A et la Tingénine B par collection après séparation par HPLC. Les composés purifiés sont resuspendus dans le DMSO puis dilués dans les tampons adéquats pour chaque test.

Exemple 2 : activité anti-inflammatoire et anti-MMP-9 de deux extraits, et des molécules purifiées de Tingénine A, Tingénine B et Célastrol

Deux extraits différents sont testés dans cet exemple, ils sont issus de culture de cellules végétales. L'extrait 2 est obtenu selon l'exemple 1, l'extrait 1 est obtenu à partir de la même culture mais avec une élicitation différente utilisant du jasmonate de méthyle et de l'acide acétylsalicylique à la place du jasmonate de méthyle et de la chitine. Les extraits 1 et 2 sont exprimés en % de chaque composant triterpène (Tingénine A : TA, Tingénine B : TB, Célastrol : CEL) par rapport à la quantité total en TA, TB et CEL dans l'extrait dans le tableau 1 ci-dessous.

[Tableau 1]

| Produits à tester (solution dans le DMSO) (μg/ml en poids sec de TA, TB et CEL) | TA | TB | CEL |
|---|---|---|---|
| CEL (49 μg/ml) | 0% | 0% | 100% |
| TA (125 μg/ml) | 100% | 0% | 0% |
| TB (125 μg/ml) | 0% | 100% | 0% |
| Extrait 1 (190 μg/ml) | 55% | 36% | 8% |
| Extrait 2 (355 μg/ml) | 27% | 23% | 50% |

Les solutions mères d'extraits en μg/ml (en poids sec de TA, TB et CEL) sont quantifiées par HPLC (par rapport aux standards étalons). Les produits à tester sont dissous dans du DMSO (solution mère). Trois concentrations sont testées, 45, 135 et 405 ng/mL, pour TA et TB. Pour le Célastrol, trois concentrations sont également testées, 15, 45 et 135 ng/ml. Pour les deux extraits, trois concentrations sont également testées, pour l'extrait 1 (45, 135 et 405 ng/ml) et pour l'extrait 2 (20, 60 et 180 ng/ml).

Un contrôle positif consiste en un inhibiteur JAK (CAS 457081-03-7 Calbiochem) testé à 10μM (dilué dans du DMSO).

La lignée cellulaire utilisée est une lignée de kératinocytes humains, cette lignée est cultivée à 37°C sous atmosphère de 5% $CO_2$.

Les kératinocytes humains sont ensemencés dans des plaques de 24 puits (75 000 cellules par puits) et cultivés pendant 48 heures dans un milieu de culture (Keratinocyte-SFM, supplémenté en Epithelial Growth Factor 0,25 ng/ml, extrait pituitaire 25 μg/ml et gentamycine 25 μg/ml). Le milieu de culture est alors remplacé par du milieu frais contenant les échantillons à tester ou non (groupe contrôle), les cellules sont à nouveau incubées pendant 24 heures (milieu d'essai : Keratinocyte-SFM, supplémenté en gentamycine 25 μg/ml). L'opération est renouvelée à nouveau en présence d'un mélange de cytokines stimulatrice des kératinocytes : IL-17, Oncostatin M et TNFα à 3ng/ml chacune. Le temps de culture est de 24 heures pour les expériences de RT-qPCR et de 48 heures pour les expériences d'ELISA. Les cellules sont ensuite lavées dans une solution PBS (Phosphate Buffered Saline) et congelées immédiatement à -80°C. Les expériences sont réalisées en triplicata.

*Analyse RT-qPCR*

L'expression des marqueurs DEFB4A, S100A7, CXCL10, CXCL11, CXCL16, CCL5, CCL20, IL-8 (CXCL8), IL-23A et MMP-9 par les kératinocytes à la suite de l'induction, est déterminée par RT-qPCR comme décrit dans la littérature avec des kits de Reverse Transcriptase (Roche) et de PCR quantitative. Deux témoins positifs de PCR (RPS28 et GAPDH, exprimés constitutivement) sont utilisés en parallèle afin de normaliser les résultats obtenus.

*Analyse ELISA des protéines marqueurs IL-8 et MMP-9*

Une seule concentration pour chaque composé a été testée dans cette expérience : TA (500 ng/ml), TB (500 ng/ml), extrait 1 (200 ng/ml) et Extrait 2 (150 ng/ml), contrôle positif (inhibiteur JAK, 10 μM). Les kératinocytes humains sont ensemencés dans des plaques de 24 puits (75 000 cellules par puits) et cultivés seulement 24 heures dans le même milieu de culture que décrit précédemment. Puis le protocole est identique au précédent, toutes les conditions

expérimentales sont réalisées en triplicata. A la fin du temps d'incubation, les surnageants sont récupérés pour la quantification des marqueurs (IL-8 et MMP-9). L'analyse des données brutes est faite avec le logiciel Microsoft Excel. La comparaison inter-groupes est réalisée par un test de Student non-apparié.

*Résultats (RT-qPCR)*

La stimulation des kératinocytes avec le mélange des cytokines IL-17, Oncostatin M et TNFα à 3ng/ml chacune, pendant 24 heures, induit un profil inflammatoire typique caractérisé par une forte up-régulation des gènes codant pour des marqueurs de l'immunité innée, et autres marqueurs inflammatoires (tableau 2).

[Tableau 2]

| | Gènes | Groupe stimulé | + JAK Inh |
|---|---|---|---|
| | | % Stimulation / Gènes témoins | |
| Peptides Anti-microbiens | DEFB4A | 500 765 | 7 |
| | S100A7 | 17 988 | 25 |
| Cytokines / Chemokines | CXCL10 | 477 | 38 |
| | CXCL11 | 405 | 14 |
| | CXCL16 | 331 | 76 |
| | CCL5 | 1 639 | 16 |
| | CCL20 | 2 065 | 292 |
| | IL-8 | 6 466 | 18 |
| | IL-23A | 843 | 67 |
| Matrice Extracellulaire | MMP-9 | 507 | 40 |

Le composé de référence (inhibiteur JAK) testé à 10μM contrecarre tous les effets de la stimulation des kératinocytes, en effet tous les gènes qui étaient up-régulés ne le sont plus en présence de cet inhibiteur. Ce résultat permet de valider ce test.

Les effets sur les marqueurs inflammatoires de la Tingénine A, la Tingénine B, du Célastrol et des deux extraits sont résumés dans le tableau 3 (effet des Tingénines A et B et du célastrol sur l'expression des gènes sur des kératinocytes humains stimulés) et le tableau 4 (effet des extraits 1 et 2 sur l'expression des gènes sur des kératinocytes humains stimulés) ci-dessous.

[Tableau 3]

| | % Stimulation / Gènes témoins | | | | | | | | |
|---|---|---|---|---|---|---|---|---|---|
| | Tingénine A (ng/ml) | | | Tingénine B (ng/ml) | | | Célastrol (ng/ml) | | |
| | 45 | 135 | 405 | 45 | 135 | 405 | 15 | 45 | 135 |
| DEFB4A | 81 | 63 | 22 | 67 | 48 | 16 | 90 | 32 | 3 |
| S100A7 | 91 | 91 | 70 | 76 | 63 | 57 | 78 | 53 | 16 |
| CXCL10 | 108 | 80 | 31 | 93 | 83 | 30 | 72 | 20 | 10 |
| CXCL11 | 107 | 85 | 23 | 78 | 57 | 27 | 59 | 5 | 1 |
| CXCL16 | 116 | 93 | 50 | 83 | 77 | 54 | 95 | 51 | 23 |
| CCL5 | 92 | 64 | 19 | 68 | 55 | 19 | 79 | 12 | 3 |
| CCL20 | 112 | 124 | 80 | 105 | 85 | 82 | 86 | 35 | 11 |
| IL-8 | 113 | 125 | 53 | 96 | 70 | 44 | 100 | 36 | 5 |
| IL-23A | 123 | 102 | 55 | 80 | 72 | 34 | 63 | 41 | 15 |
| MMP-9 | 98 | 93 | 34 | 88 | 63 | 32 | 139 | 88 | 9 |

[Tableau 4]

| | % Stimulation / Gènes témoins | | | | | |
|---|---|---|---|---|---|---|
| Gènes | Extrait 1 (ng/ml) | | | Extrait 2 (ng/ml) | | |
| | 45 | 135 | 405 | 20 | 60 | 180 |
| DEFB4A | 70 | 24 | 3 | 79 | 37 | 8 |
| S100A7 | 114 | 62 | 17 | 104 | 55 | 26 |
| CXCL10 | 68 | 26 | 7 | 68 | 29 | 12 |
| CXCL11 | 65 | 16 | 1 | 52 | 23 | 1 |
| CXCL16 | 104 | 57 | 27 | 91 | 60 | 33 |
| CCL5 | 79 | 17 | 3 | 69 | 25 | 5 |
| CCL20 | 120 | 62 | 10 | 84 | 52 | 17 |
| IL-8 | 139 | 66 | 7 | 94 | 59 | 18 |
| IL-23A | 162 | 90 | 41 | 134 | 68 | 38 |
| MMP-9 | 136 | 56 | 8 | 128 | 60 | 19 |

Dans ces conditions expérimentales, la Tingénine A et la Tingénine B testées à 45, 135 et 405 ng/ml montrent une down-régulation de ces gènes et de façon concentration-dépendante sur l'ensemble des marqueurs évalués. Les effets des Tingénines A et B sont particulièrement importants avec la concentration testée la plus forte. De même, les effets des deux extraits testés diminuent de façon concentration-dépendante l'expression des marqueurs évalués lors de ce test.

Les inventeurs mettent ainsi en évidence que les triterpènes présents dans les deux extraits présentent un effet anti-Th17, principalement aux deux plus fortes concentrations.

*Résultats (ELISA d'IL-8 et MMP-9)*

Les effets de la Tingénine A, la Tingénine B, du Célastrol et des deux extraits sur la libération d'IL-8 et de MMP-9 sont résumés dans le tableau 5 ci-dessous (* P<0,05 ; ** P<0,01 ; *** P<0,001 vs contrôle).

[Tableau 5]

| Groupes/ Conditions | Libération d'IL-8 (pg/ml) ± sem | Libération de MMP-9 (pg/ml) ± sem |
|---|---|---|
| Non-stimulé | 89 ± 14 | 252 ± 6 |
| Contrôle | 7 354 ± 113 | 1 077 ± 50 |
| Inhibiteur JAK (10 μM) | 2 178 ± 225 *** | 389 ± 18 *** |
| Tingénine A (500 ng/ml) | 3 305 ± 170 *** | 787 ± 105 |
| Tingénine B (500 ng/ml) | 3 213 ± 145 *** | 828 ± 31 * |
| Célastrol (100 ng/ml) | 83 ± 7 *** | 395 ± 15 *** |
| Extrait 1 (200 ng/ml) | 819 ± 94 *** | 609 ± 6 *** |
| Extrait 2 (150 ng/ml) | 424 ± 18 *** | 750 ± 38 ** |

Dans les conditions non stimulées, un faible niveau d'IL-8 et de MMP-9 sont sécrétés par les kératinocytes. La stimulation des kératinocytes par le mélange des cytokines résulte en une forte libération d'IL-8 et de MMP-9. L'inhibiteur JAK (10 μM), choisi comme composé de référence, inhibe fortement et statistiquement la libération de ces deux marqueurs. Ces résultats attendus permettent de bien valider ce test.

Dans ces conditions expérimentales, la Tingénine A (500 ng/ml), la Tingénine B (500 ng/ml), le Célastrol (100 ng/ml), l'extrait 1 (200 ng/ml) et l'extrait 2 (150 ng/ml) montrent tous clairement un effet inhibiteur sur la libération d'IL-8 et de MMP-9 dues à la stimulation des kératinocytes. Les deux extraits et le célastrol montrent une activité anti-inflammatoire plus puissante comparée à celle des Tingénines A et B.

Les inventeurs démontrent ainsi dans un modèle de kératinocytes humains stimulés par un mélange de cytokines que les extraits 1 et 2 et les triterpènes pentacycliques présents dans ces extraits et testés seuls présentent un effet anti-Th17 au niveau de l'expression des gènes et que cet effet est confirmé au niveau protéique. Il est important de noter que les extraits contenant des quantités modulables des trois triterpènes pentacycliques ont des activités inhibitrices (Th17) encore plus fortes. De plus, tous ces composés seuls et les extraits testés sont capables d'inhiber la MMP-9 qui est l'une des principales cibles du traitement du vitiligo.

Exemple 3 : activité antioxydante des deux extraits, et des molécules purifiées de Tingénine A, Tingénine B et Célastrol

Le stress oxydatif fait partie des causes possibles de l'apparition du vitiligo. Cette expérience a pour objectif de mesurer l'activité antioxydante de chaque triterpène pentacyclique et des deux extraits, en comparaison avec un témoin positif bien connu, la vitamine C. La méthode *in tubo* utilisée est celle d'évaluation ORAC (Oxygen Radical Antioxydant Capacity) adaptée de celle décrite par Davalos et al. (J Agric Food Chem 52(1):48-54, 2004). L'indice ORAC permet d'évaluer la capacité anti-oxydante de chaque composé testé dans l'exemple 2. Ce test permet d'évaluer et de classer les différents composés testés suivant leur pouvoir anti-oxydant vis-à-vis des radicaux libres péroxyl. Le AAPH (2,2'-azobis-2-méthyl-propanimidamide, dihydrochloride) est à l'origine de radicaux libres péroxyl. Ce test est utilisé pour mimer la cinétique de dégradation de la fluorescéine par les radicaux libres. Ceci se traduit par une diminution de fluorescence en fonction du temps et de la concentration en actif anti-oxydant présent dans l'extrait testé. L'acide 3,4-dihydro-6-hydroxy-2,5,7,8-tétraméthyl-2H-1-benzopyran-2-carboxylique ou Trolox est utilisé comme standard, il s'agit d'un analogue hydrophile de la vitamine E (anti-oxydant connu). L'ajout de Trolox (gamme de référence) ou d'extrait ou triterpène pentacyclique, protège la fluorescéine de la dégradation. Ceci permet d'évaluer une activité antioxydante mesurée par rapport à une gamme de Trolox.

Toutes les solutions sont réalisées dans du tampon phosphate 75 mM, pH à 7,6. La fluorescéine est à 1,17 μM (solution mère 117 mM). AAPH à 125 mM est préparé extemporanément, Trolox à 1 mM. Dans une plaque de 96 puits noir, il est déposé 20 μl d'antioxydant (gamme Trolox ou composé à tester), 160 μl de fluorescéine à 1,17 μM. la plaque est recouverte d'un film, puis est incubée pendant 15 minutes à 37°C. la solution de AAPH est ajoutée manuellement et le plus rapidement possible à raison de 20 μl par puits. La plaque est alors incubée immédiatement à 37°C dans un spectrofluorimètre (SpectraMax). Une lecture est enregistrée toutes les minutes pendant 90 minutes. La longueur d'onde d'excitation est de 485 nm et la longueur d'onde d'émission est de 520 nm. Chaque essai est réalisé en triplicata.

Les aires sous la courbe (AUC) pour chacun des essais sont calculées. Les AUC nets sont déterminées de la façon suivante :

$$\text{AUC net} = \text{AUC point de gamme ou échantillon} - \text{AUC blanc.}$$

La droite d'étalonnage est établie, la concentration en Trolox (μM) en fonction de l'AUC net. L'équation de la droite est utilisée pour déterminer un équivalent en Trolox (μM) pour chacun des échantillons dosés.

Eq Trolox en μM = a x (AUC net)2 + b x (AUC net), a et b sont déterminés par l'équation de la droite.

Pour être dosés, les échantillons sont dilués, de sorte que les valeurs mesurées soient comprises dans la gamme étalon utilisée pour le dosage. L'acide ascorbique (vitamine C) est utilisé comme référence.

L'indice ORAC correspond à des %moles de Trolox / 100g de matières.

Les résultats ci-dessous ont été calculés pour 100g d'extrait en Triterpènes, Tingénine A, Tingénine B et célastrol, soit le calcul :

Indice ORAC = eq Trolox en μM X 20 (prise d'essai) x (100 000 / [extrait testé μg/ml] x 20 (prise d'essai)).

*Résultats*

Les résultats de cette expérience en termes d'activité antioxydante et de comparaison d'indice ORAC / 100g en Triterpènes sont présentés dans le tableau 6 ci-dessous.

[Tableau 6]

| Composé (μg/ml) | Indice ORAC / 100g triterpènes (x 1000) | Ecart-type Indice ORAC / 100g triterpènes (x 1000) |
|---|---|---|
| Vitamine C (3) | 69 813 | ± 32 850 |
| Extrait 1 (1,5) | 1 085 333 | ± 46 533 |
| Extrait 2 (6) | 245 600 | ± 12 533 |
| Célastrol (30) | 126 466 | ± 13 066 |
| Tingénine A (0,75) | 33 960 | ± 21 733 |
| Tingénine B (7,5) | 124 320 | ± 49 733 |

Ainsi, plus l'indice ORAC est élevé plus l'activité antioxydante est importante. Dans l'ordre décroissant (indice ORAC/100 g en Triterpènes) : Extrait 1 > Extrait 2 > Célastrol ≥ Tingénine B > Vitamine C > Tingénine A.

Les inventeurs démontrent que dans ces conditions expérimentales, les extraits 1 et 2 ont un pouvoir anti-oxydant supérieur à la référence (vitamine C), et que les extraits présentent une activité antioxydante plus forte que les triterpènes pris isolément. Seule la Tingénine A présente un pouvoir anti-oxydant inférieur à la vitamine C.

En conclusion, les inventeurs démontrent de manière surprenante, que non seulement les extraits comprenant des Triterpènes (Tingénines A et B, Célastrol) ont une activité anti-Th17 forte mais sont également dotés de propriétés antioxydantes importantes. Ces deux effets se conjuguent et s'avèrent très bénéfiques pour préserver les fonctions normales des mélanocytes et des kératinocytes, pour prévenir, stabiliser la progression du vitiligo et potentiellement revenir à une pigmentation.

## Revendications

1. Composition comprenant la Tingénine A, la Tingénine B et le Célastrol ou leurs sels pharmaceutiquement acceptables pour son utilisation dans le traitement et/ou la prévention du vitiligo.

2. Composition pour son utilisation selon la revendication 1, dans laquelle la Tingénine A, la Tingénine B et le Célastrol ou leurs sels pharmaceutiquement acceptables sont présents au sein d'un extrait d'une plante de la famille des Celastraceae.

3. Composition pour son utilisation selon la revendication 2, dans laquelle la plante de la famille des Celastraceae est choisi parmi *Tripterygium wilfordii, Tripterygium hypoglaucum* et *Celastrus orbiculatus.*

4. Composition pour son utilisation selon la revendication 2 ou 3, dans laquelle l'extrait est un extrait brut enrichi obtenu par le procédé suivant :

   (i) une phase de prolifération de cellules d'une plante de la famille des Celastraceae dans un milieu de prolifération,
   (ii) une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
   (iii) la préparation d'un extrait brut enrichi en Tingénine A, Tingénine B et Célastrol à partir de la culture cellulaire obtenue à l'étape (ii).

5. Composition pour son utilisation selon la revendication 4, comprenant entre 0,01 et 2% d'extrait brut enrichi en poids par rapport au poids total de la composition.

6. Composition pour son utilisation selon la revendication 4 ou 5, dans laquelle l'extrait brut enrichi comprend entre 90% et 100% de Tingénine A, Tingénine B et Célastrol en poids par rapport au poids total de l'extrait brut enrichi.

7. Composition pour son utilisation selon l'une quelconque des revendications 4 à 6, dans laquelle l'extrait brut enrichi comprend :

   entre 1 et 20%, notamment entre 5 et 15 %, notamment entre 8 et 12 % de Tingénine A en poids par rapport

au poids total de l'extrait,
entre 1 et 20 %, notamment entre 5 et 15 %, notamment entre 8 et 12 % de Tingénine B en poids par rapport au poids total de l'extrait, et
au moins 60 %, notamment au moins 80% de Célastrol en poids par rapport au poids total de l'extrait.

**8.** Composition pour son utilisation selon l'une quelconque des revendications 1 à 7, **caractérisée en ce qu'**elle se présente sous une forme propre à une application topique.

**9.** Composition pour son utilisation selon l'une quelconque des revendications 1 à 8, dans laquelle le vitiligo est médié par les cytokines et les chémokines de type Th17.

**10.** Composition pour son utilisation selon la revendication 9, dans laquelle les cytokines Th17 induisent de inhibitions de la production de la mélanine par les mélanocytes.

**11.** Composition pour son utilisation selon la revendication 9 ou 10, dans laquelle la protéine MMP-9 est surexprimée par les kératinocytes.

**Patentansprüche**

**1.** Zusammensetzung, umfassend Tingenin A, Tingenin B und Celastrol oder deren pharmazeutisch akzeptable Salze für ihre Verwendung zur Behandlung und/oder Vorbeugung von Vitiligo.

**2.** Zusammensetzung für ihre Verwendung nach Anspruch 1, wobei das Tingenin A, das Tingenin B und das Celastrol oder deren pharmazeutisch akzeptablen Salze in einem Extrakt einer Pflanze aus der Familie der Celastraceae vorhanden sind.

**3.** Zusammensetzung für ihre Verwendung nach Anspruch 2, wobei die Pflanze aus der Familie der Celastraceae aus *Tripterygium wilfordii, Tripterygium hypoglaucum* und *Celastrus orbiculatus* ausgewählt ist.

**4.** Zusammensetzung für ihre Verwendung nach Anspruch 2 oder 3, wobei der Extrakt ein angereicherter Rohextrakt ist, der durch das folgende Verfahren erhalten wird:

(i) eine Phase des Wachstums von Zellen einer Pflanze aus der Familie Celastraceae in einem Wachstumsmedium,
(ii) eine Phase der Elicitation durch Hinzufügen eines Elicitationscocktails in die in Schritt (i) erhaltene Zellkultur, wobei der Elicitationscocktail mindestens einen Elicitator vom Typ Monocarbonsäureverbindung und mindestens einen biotischen Elicitator umfasst, und
(iii) die Zubereitung eines mit Tingenin A, Tingenin B und Celastrol angereicherten Rohextrakts aus der in Schritt (ii) erhaltenen Zellkultur.

**5.** Zusammensetzung für ihre Verwendung nach Anspruch 4, umfassend zwischen 0,01 und 2 Gew.-% angereicherten Rohextrakt im Verhältnis zum Gesamtgewicht der Zusammensetzung.

**6.** Zusammensetzung für ihre Verwendung nach Anspruch 4 oder 5, wobei der angereicherte Rohextrakt zwischen 90 und 100 Gew.-% Tingenin A, Tingenin B und Celastrol im Verhältnis zum Gesamtgewicht des angereicherten Rohextrakts umfasst.

**7.** Zusammensetzung für ihre Verwendung nach einem der Ansprüche 4 bis 6, wobei der angereicherte Rohextrakt umfasst:

zwischen 1 und 20 Gew.-%, insbesondere zwischen 5 und 15 Gew.-%, insbesondere zwischen 8 und 12 Gew.-% Tingenin A im Verhältnis zum Gesamtgewicht des Extrakts,
zwischen 1 und 20 Gew.-%, insbesondere zwischen 5 und 15 Gew.-%, insbesondere zwischen 8 und 12 Gew.-% Tingenin B im Verhältnis zum Gesamtgewicht des Extrakts, und
mindestens 60 Gew.-%, insbesondere mindestens 80 Gew.-% Celastrol im Verhältnis zum Gesamtgewicht des Extrakts.

**8.** Zusammensetzung für ihre Verwendung nach einem der Ansprüche 1 bis 7, **dadurch gekennzeichnet, dass** sie in einer Form vorliegt, die für eine topische Anwendung geeignet ist.

**9.** Zusammensetzung für ihre Verwendung nach einem der Ansprüche 1 bis 8, wobei die Vitiligo durch die Zytokine und die Chemokine vom Typ Thl7 vermittelt wird.

**10.** Zusammensetzung für ihre Verwendung nach Anspruch 9, wobei die Zytokine Thl7 Hemmungen der Produktion von Melanin durch die Melanozyten induzieren.

**11.** Zusammensetzung für ihre Verwendung nach Anspruch 9 oder 10, wobei das Protein MMP-9 durch die Keratinozyten überexprimiert wird.

## Claims

**1.** A composition comprising Tingenin A, Tingenin B and Celastrol or their pharmaceutically acceptable salts for its use in the treatment and/or prevention of vitiligo.

**2.** The composition for its use according to claim 1, wherein Tingenin A, Tingenin B and Celastrol or their pharmaceutically acceptable salts are present in an extract of a plant of the Celastraceae family.

**3.** The composition for its use according to claim 2, wherein the plant of the Celastraceae family is chosen from *Tripterygium wilfordii, Tripterygium hypoglaucum* and *Celastrus orbiculatus.*

**4.** The composition for its use according to claim 2 or 3, wherein the extract is an enriched crude extract obtained by the following method:

(i) a proliferation phase of cells of a plant of the Celastraceae family in a proliferation medium,
(ii) an elicitation phase by adding an elicitation cocktail to the cell culture obtained in step (i), said elicitation cocktail comprising at least one elicitor of the monocarboxylic compound type and at least one biotic elicitor, and
(iii) the preparation of a crude extract enriched in Tingenin A, Tingenin B and Celastrol from the cell culture obtained in step (ii).

**5.** The composition for its use according to claim 4, comprising between 0.01 and 2% of enriched crude extract by weight relative to the total weight of the composition.

**6.** The composition for its use according to claim 4 or 5, wherein the enriched crude extract comprises between 90% and 100% of Tingenin A, Tingenin B and Celastrol by weight relative to the total weight of the enriched crude extract.

**7.** The composition for its use according to any one of claims 4 to 6, wherein the enriched crude extract comprises:

between 1 and 20%, in particular between 5 and 15%, in particular between 8 and 120 of Tingenin A by weight relative to the total weight of the extract,
between 1 and 20%, in particular between 5 and 15%, in particular between 8 and 120 of Tingenin B by weight relative to the total weight of the extract, and
at least 60%, in particular at least 80% of Celastrol by weight relative to the total weight of the extract.

**8.** The composition for its use according to any one of claims 1 to 7, **characterized in that** it is in a form suitable for topical application.

**9.** The composition for its use according to any one of claims 1 to 8, wherein vitiligo is mediated by Thl7 type cytokines and chemokines.

**10.** The composition for its use according to claim 9, wherein the Thl7 cytokines induce inhibition of the production of melanin by melanocytes.

**11.** The composition for its use according to claim 9 or 10, wherein the MMP-9 protein is overexpressed by keratinocytes.

[Fig. 1]

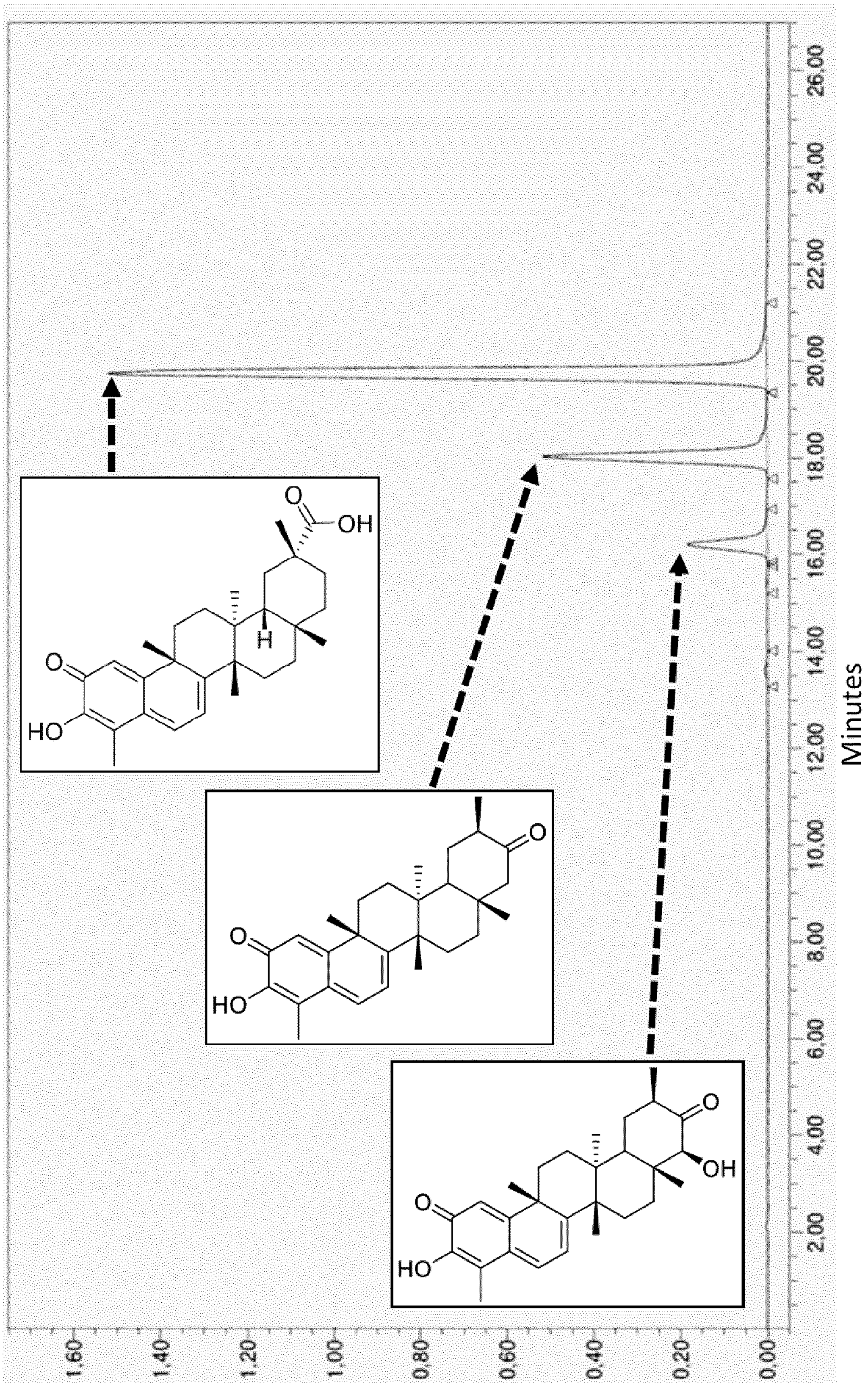
Minutes
2,00
4,00
6,00
8,00
10,00
12,00
14,00
16,00
18,00
20,00
22,00
24,00
26,00
0,00
0,20
0,40
0,60
0,80
1,00
1,20
1,40
1,60

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **BOUKHEDOUNI et al.** *Pigment Cell Melanoma Research,* 2018, vol. 31 (6), 756-757
- **KOTOBUKI et al.** *Pigment Cell Melanoma Research,* 2012, vol. 25 (2), 219-230
- **COPPEDE et al.** *Plant Cell Tiss Organ Cuit,* 2014, vol. 118, 33-43
- *CHEMICAL ABSTRACTS,* 457081-03-7
- **DAVALOS et al.** *J Agric Food Chem,* 2004, vol. 52 (1), 48-54